(19) Europäisches Patentamt
European Patent Office
Office européen des brevets

(11) **EP 2 113 249 A1**

(12) **EUROPEAN PATENT APPLICATION**
published in accordance with Art. 153(4) EPC

(43) Date of publication:
**04.11.2009 Bulletin 2009/45**

(51) Int Cl.:
*A61K 9/70* (2006.01)    *A61K 31/137* (2006.01)
*A61K 47/32* (2006.01)    *A61P 31/10* (2006.01)

(21) Application number: **08711853.5**

(22) Date of filing: **22.02.2008**

(86) International application number:
**PCT/JP2008/053085**

(87) International publication number:
**WO 2008/102880 (28.08.2008 Gazette 2008/35)**

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **23.02.2007  PCT/JP2007/053366**

(71) Applicant: **Nichiban Co. Ltd.
Tokyo 112-8663 (JP)**

(72) Inventors:
• **KAWAHARA, Koji
Tokyo 112-8663 (JP)**
• **SHIMADA, Noriko
Tokyo 112-8663 (JP)**

(74) Representative: **HOFFMANN EITLE
Patent- und Rechtsanwälte
Arabellastrasse 4
81925 München (DE)**

(54) **ANTIMYCOTIC PATCH**

(57)    A nail and/or skin patch for preventing or treating mycoses is provided that is able to maintain a drug concentration in the nails and/or skin horny layer at a high concentration for a long period of time without adding a dissolving agent that prevents precipitation of drug crystals or permeation enhancer that promotes penetration of drug into the nails and/or skin, retains superior adhesion even when adhered for a long period of time and has causes little skin irritation.

A nail and/or skin patch for preventing or treating mycoses, which contains an antimycotic, having an octanol/water partition coefficient in the form of a logKo/w value of 4 or more, in a dissolved state in an acrylic-based pressure-sensitive adhesive layer or silicone-based pressure-sensitive adhesive layer.

**Fig. 1**

EP 2 113 249 A1

**Description**

TECHNICAL FIELD

The present invention relates to a transdermal patch for preventing and/or treating mycotic infections (referred to as mycoses) of the nails and/or skin. More particularly, it relates to a highly therapeutically effective transdermal patch having satisfactory drug permeability with respect to the nails and horny layer.

BACKGROUND ART

While typical examples of mycoses affecting numerous Japanese include tinea, dermal candidiasis and pygalgia, tinea is the most common of these, with tinea infecting one in four Japanese. Moreover, one in eight Japanese become complicated with tinea unguium, which is difficult to treat once it has occurred, and the incidence of tinea is said to be one of every two Japanese among Japanese age 60 and older.

Accompanying dramatic advances made in the field of antimycotics in recent years, treatment of tinea, which is the most important disease among mycoses, has improved remarkably. However, although the activity of a drug itself is high, in the case of applying a drug in the form of an externally applied preparation such as an ointment, cream or liquid, numerous problems in terms of actual treatment have been indicated to occur, including difficulty in retaining the drug at an affected area for a sufficient amount of time for allowing medicinal effects to be demonstrated, and patient substantially failing to administer regularly the drug several times per day. In addition, although treatment of mycoses in the field of dermatology is considered to require persistent treatment over an extended period of time, with respect to tinea unguium in particular, which involves a local mycosis of the nails and nail beds, it is currently difficult to cure completely for reasons such as those described above.

Although treatment by long-term oral administration is currently employed for the treatment of tinea unguium, oral antimycotics have problems such as impairment of liver function, and numerous other adverse side effects caused by long-term oral administration. In addition, the incidence of tinea unguium among diabetes patients is known to be comparatively high, and in the case of having been previously diagnosed or treated for diabetes, there is a high likelihood of a plurality of therapeutic drugs being administered, thereby frequently making oral administration of antimycotics for treatment of tinea unguium difficult.

On the other hand, treatment methods using a transdermal patch not only solve the problems of externally applied preparations and oral administration as described above, but also lead to a reduction in the number of administrations since medicinal effects are sustained for a long period of time. In particular, since administration is easy since the patch is only required to be adhered to the skin, it also offers advantages for the improvement of compliance and easier starting and discontinuation of administration. Moreover, although changes in blood drug concentration caused by diet must be taken into consideration in the case of oral administration, transcutaneous absorption is not affected by diet, thereby also offering the advantage of administration without concern over dosage.

In consideration of these circumstances, transcutaneous absorption patches are expected for patients suffering from tinea, which increases in incidence with age, and particularly patients suffering from tinea unguium, to provide a useful drug administration method that is convenient, safe and effective.

Since fungi mainly proliferate by infiltrating the horny layer of the skin, conditions for an antimycotic to demonstrate superior medicinal effects against mycoses such as tinea include the drug itself having potent antimycotic activity and the sustainability of drug concentration at a high level for a long period of time at sites of infection in the skin horny layer and nails. Recently, expectations have been placed on the development of transdermal patch enabling the sustained administration of a drug to an affected area for a long period of time.

However, since many antimycotics are water-soluble and are extremely insoluble in the patch base, even if an antimycotic is incorporated in a patch, there are many cases in which crystals of the antimycotic end up precipitating on the surface of the patch. As a result of crystals of antimycotic precipitating in this manner, together with the release of antimycotic from the patch decreasing, adhesion to the nails and/or skin decreases, thereby causing a decrease in the transfer of antimycotic to the nails and/or skin, and making it difficult for the drug to be efficiently and continuously absorbed. On the other hand, since nails are 100 to 200 times thicker than the horny layer, it is necessary to contain a sufficient amount of drug in the patch. For these reasons, there have been virtually no patches thus far capable of allowing a sufficient concentration of an active ingredient in the form of an antimycotic to reach a target site, and topical treatment of tinea unguium has typically been ineffective.

Consequently, there has been a need for a method for treating mycoses, including tinea unguium, capable of maintaining a high concentration of antimycotic in the nails and skin while also allowing an effective amount of drug to be administered locally to nail beds in particular.

In order to solve previous problems, a transdermal patch, in which a dissolving agent has been added to prevent precipitation of drug crystals, has been developed as a transdermal patch that improves the solubility of various antimycotics such as terbinafine hydrochloride into a base (Patent Document 1), and a patch containing a permeation enhancer has been developed as a transdermal patch that improves permeation and absorption of various antimycotics such as terbinafine hydrochloride into nails (Patent Documents 2 and 3).

With respect to the patch containing a permeation enhancer, since substances such as aliphatic alcohols and fatty acids used as permeation enhancers demonstrate their effects by permeating through the skin, there are many instances in which they cause skin irritation (Non-Patent Documents 1 and 2), thus making it desirable to design formulations that do not use permeation enhancers. However, since the skin permeability of the drug itself is insufficient in nearly all cases, there are many cases in which the incorporation of permeation enhancer is required. Even in the case of having incorporated a permeation enhancer, although transfer of drug to the nails is high, transfer of drug to the nail bed was not satisfactory.

On the other hand, formulations using a dissolving agent as described above are primarily used for adhesion to skin, and since they are not developed as a formulation able to be adhered to nails for the purpose of treatment and/or prevention of tinea unguium, they do not solve the previous problems.

In addition, treatment of tinea unguium by topical administration has the shortcoming of it being difficult for the drug to migrate to the nail bed as a result of permeability of the drug being inhibited by the nail plate which is a thick, hard keratin layer. In addition, it is also difficult to control adhesion to the nails and/or skin as a result of incorporating additives such as dissolving agent and permeation enhancers, while also having the disadvantage of causing skin irritation. Moreover, since these additives are in the form of liquids or oils, there is the additional shortcoming of the additives bleeding out of the patch or being susceptible to changes in adhesive properties as a result of sudden changes in the storage temperature of the patch or storing the patch for a long period of time. In addition, the phenomenon of "residual adhesive" may occur when the preparation is peeled off depending on the added amount.

Patent Document 1: Japanese Unexamined Patent Publication No. 2002-363070
Patent Document 2: Japanese Unexamined International
Patent Publication No. 2003-525641
Patent Document 3: Japanese Unexamined International
Patent Publication No. 2005-501885
Non-Patent Document 1: Tanojo H. et al.: In vitro human barrier modulation by topical application of fatty acids, Skin Pharmacol. Appl. Skin Physiol., 11(2), 1998
Non-Patent Document 2: Kanikkannan N. et al.: Skin permeation enhancement effect and skin irritation of saturated fatty alcohols, 6, 2002

DISCLOSURE OF THE INVENTION

Problems to be Solved by the Invention

For the reasons indicated above, there has been a demand for the providing of a patch for preventing and treating mycoses of the nails and/or skin capable of allowing an effective amount of a drug to be topically administered to a target site with minimal systemic exposure while maintaining a high drug concentration for a long period of time in the nails and/or skin horny layer, having superior adhesion even if adhered for a long period of time, and causing little skin irritation. Means for Solving the Problems

As a result of conducting extensive studies to solve the previous problems, the inventors of the present invention found that by using an acrylic-based pressure-sensitive adhesive or silicone-based pressure-sensitive adhesive as a base and incorporating in the base an active ingredient in the form of an antimycotic having a logKo/w value representing the octanol/water partition coefficient of 4 or more in a patch for the purpose of preventing and treating mycoses of the nails and/or skin, a patch can be obtained that is capable of maintaining a high drug concentration in the nails and/or skin horny layer over a long period of time without having to add a dissolving agent that prevents precipitation of drug crystals or permeation enhancer that promotes penetration of drug into the nails and/or skin, has superior adhesion even when adhered for a long period of time, and causes little skin irritation, thereby leading to completion of the present invention.

Namely, the present invention relates to a patch for the nails and/or skin for preventing or treating mycoses, which contains an antimycotic, having an octanol/water partition coefficient in the form of a logKo/w value of 4 or more, in a dissolved state in an acrylic-based pressure-sensitive adhesive layer or silicone-based pressure-sensitive adhesive layer.

Effects of the Invention

In the patch for the nails and/or skin for prevention or treatment of mycoses in the present invention, as a result of using an acrylic-based pressure-sensitive adhesive or silicone-based pressure-sensitive adhesive as a base, and containing an antimycotic having a logKo/w value, which represents an octanol/water partition coefficient, of 4 or more as the active ingredient thereof, the antimycotic is dissolved at a high concentration in the acrylic-based pressure-sensitive adhesive or silicone-based pressure-sensitive adhesive. Consequently, since it is not necessarily required to add a dissolving agent that prevents precipitation of drug crystals or permeation enhancer that promotes penetration of drug into the nails and/or skin to the patch, control of adhesion of the patch is easy, and adhesion of the patch to the nails and/or skin can be maintained for a long period of time. In addition, a stable patch can be provided since bleeding of a dissolving agent or permeation enhancer from the patch or changes in properties and the like, caused by sudden changes in the patch storage temperature or storing for a long period of time, can be prevented.

In this manner, in the patch for nails and/or skin in the present invention, as a result of containing an antimycotic having a logKo/w value, which represents the octanol/water partition coefficient, of 4 or more as an active ingredient thereof at a high concentration in a dissolved state in an acrylic-based pressure-sensitive adhesive or silicone-based pressure-sensitive adhesive, a high concentration of antimycotic is able to transfer to the nails and/or skin, thereby eliminating the problem of topical products for tinea unguium in the form of decreased penetration rate of the antimycotic to the nail bed, and allowing the obtaining of high effects against mycoses such as tinea unguium in particular.

Thus, the patch in the present invention is able to demonstrate sufficient effects for the treatment of mycoses of the nails and skin, and is particularly useful in the treatment of superficial mycoses such as athlete's foot and tinea.

BRIEF DESCRIPTION OF THE DRAWINGS

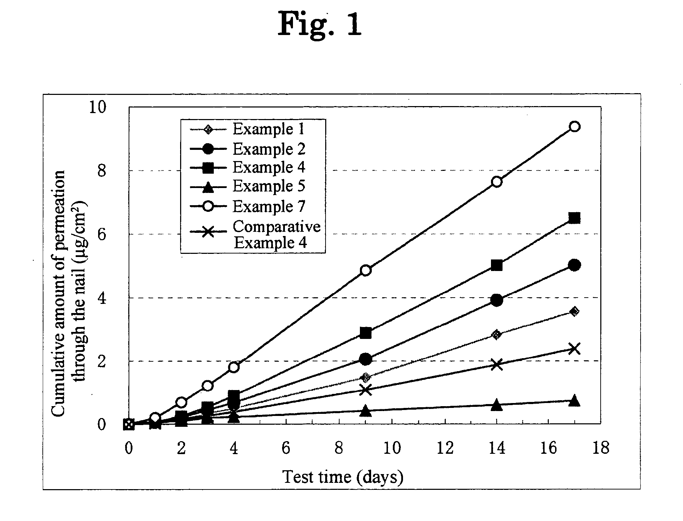

Fig. 1 shows the cumulative amount of terbinafine-base (free form) permeation through the nail.

BEST MODE FOR CARRYING OUT THE INVENTION

As will be subsequently explained in detail, the patch for nails and/or skin for preventing or treating mycoses in the present invention is a patch containing an antimycotic, having an octanol/water partition coefficient in the form of an logKo/w value of 4 or more, in a dissolved state in an acrylic-based pressure-sensitive adhesive layer or silicone-based pressure-sensitive adhesive layer.

In the present invention, logKo/w is determined in the following manner. First, a drug is dissolved in a suitable amount of octanol (or water) at 32°C followed by the further addition of an equal amount of water (or octanol) and mixing well. After the mixture divides into two layers, the concentration of drug in each layer is measured by HPLC and logKo/w is determined using the equation below.

$$\texttt{LogKo/w = log(drug concentration in octanol phase/drug}$$

$$\texttt{concentration in aqueous phase)}$$

In addition, in the patch in the present invention, a "dissolved state" refers to a state in which crystals and powders of the antimycotic are not observed and the pressure-sensitive adhesive layer is transparent in the case of visually observing the pressure-sensitive adhesive layer under conditions of a temperature of 25°C.

In the patch in the present invention, the acrylic-based pressure-sensitive adhesive layer or silicone-based pressure-sensitive adhesive layer containing the antimycotic can be formed on a backing layer or release liner. Although the thickness of the acrylic-based pressure-sensitive adhesive or silicone-based pressure-sensitive adhesive can be suitably determined as necessary, it can be specifically 10 to 200 $\mu$m and preferably 20 to 100 $\mu$m.

Antimycotic

In the antimycotic incorporated in the patch in the present invention, the logKo/w value, which represents the octanol/water partition coefficient, is 4 or more. This partition coefficient logKo/w can be calculated by using the formula indicated above, and is a value that represents the lipophilicity of a drug. In the patch in the present invention, by incorporating an antimycotic having a logKo/w value of 4 or more in an acrylic-based pressure-sensitive adhesive or silicone-based pressure-sensitive adhesive, the antimycotic can be stably maintained in a state of being dissolved at a high concentration in the pressure-sensitive adhesive layer, while also enabling transfer of a high concentration of the antimycotic into the nails and skin.

A specific example of an antimycotic having a partition coefficient logKo/w value of 4 or more includes terbinafine-base. The logKo/w value of terbinafine-base, as calculated according to the logKo/w calculation formula indicated above, is 5.8.

In addition, while acid addition salts of terbinafine (such as hydrochlorides, hydrofumarates or naphthaline-1,5-disulfonates) have logKo/w values of about 1, these acid addition salts can be incorporated in the patch in the present invention by incorporating in the patch together with a base to convert to terbinafine-base having a logKo/w value of 4 or more. Examples of bases able to be used in such cases include sodium hydroxide, potassium hydroxide, calcium hydroxide, magnesium hydroxide, sodium carbonate, sodium hydrogencarbonate, phosphates, borates, acetates, ammonia, dialkylamines, trialkylamines, tris(hydroxymethyl)aminomethane, monoethanolamine, diethanolamine, triethanolamine, isopropanolamine, diisopropanolamine and triisopropanolamine.

In the case of converting an acid addition salt of terbinafine to the free form with base as described above, the amount of base used based on the amount of terbinafine acid addition salt is at least 0.01 (weight ratio) and preferably an amount equal to a ratio of 0.01 to 1 (weight ratio) based on 1 part terbinafine acid addition salt, and this amount can be suitably determined according to the types of terbinafine acid addition salt and base. More specifically, a terbinafine acid addition salt can be converted to terbinafine-base by mixing a terbinafine acid addition salt into an acrylic-based pressure-sensitive adhesive or silicone-based pressure-sensitive adhesive, and further adding the aforementioned base at a ratio of 0.01 to 1 (weight ratio) to 1 part terbinafine acid addition salt. In this case, the base can be added to the pressure-sensitive adhesive liquid, in a crystalline form or powder form, or can be added to a pressure-sensitive adhesive solution by dissolving or dispersing crystals or powder in a suitable organic solvent, and there are no particular limitations thereon.

Acrylic-Based Pressure-Sensitive Adhesive Layer or Silicone-Based Pressure-Sensitive Adhesive Layer

A pressure-sensitive adhesive component in the form of an acrylic-based pressure-sensitive adhesive or silicone-based pressure-sensitive adhesive is incorporated in the pressure-sensitive adhesive layer in the present invention. In the patch for nails and/or skin in the present invention, any arbitrary acrylic-based pressure-sensitive adhesive normally used in patches for nails and/or skin can be used, and the acrylic-based pressure-sensitive adhesive preferably has for a base thereof a (meth)acrylic acid ester copolymer containing as an essential monomer component thereof a (meth) acrylic acid alkyl ester in which the number of carbon atoms of the alkyl group is 4 to 12. Examples of this (meth)acrylic acid alkyl ester monomer component include n-butyl acrylate, n-hexyl acrylate, n-octyl acrylate, 2-ethylhexyl acrylate, isooctyl acrylate, isononyl acrylate, n-decyl acrylate or isodecyl acrylate, and n-decyl methacrylate, isodecyl methacrylate or lauryl methacrylate as methacrylic acid esters.

Among said (meth)acrylic acid alkyl ester monomer components, 2-ethylhexyl acrylate, n-octyl acrylate, isooctyl acrylate, isononyl acrylate and lauryl methacrylate are particularly preferable.

In this (meth)acrylic acid ester copolymer, in addition to (meth)acrylic acid alkyl esters indicated above, vinyl monomers having a functional group, for example, can also be preferably incorporated as other monomer components, specific examples of which include monomers having a hydroxyl group such as 2-hydroxyethyl acrylate, 3-hydroxypropyl acrylate or 4-hydroxybutyl acrylate; monomers having a carboxyl group such as acrylic acid, methacrylic acid, maleic acid, maleic anhydride, itaconic acid or monobutyl maleate; monomers having an amino group such as acrylamide, dimethylacrylamide, diethylacrylamide, methacrylamide or N-methylolacrylamide; and, monomers having an epoxy group such as glycidyl acrylate or glycidyl methacrylate.

These vinyl monomers having a functional group can be used alone or as a combination of two or more types thereof.

The copolymerization ratio of these other monomer components, such as vinyl monomers having a functional group, in the (meth)acrylic acid ester copolymer is preferably 20% by weight or less and more preferably 10% by weight or less based on the total weight of the copolymer.

Other containable monomer components can be further incorporated in the (meth)acrylic acid ester copolymer in addition to the other monomer components indicated above. Examples of such components include vinyl esters such as vinyl acetate, unsaturated nitriles such as acrylonitrile or methacrylonitrile, and vinyl aromatic compounds such as styrene. Moreover, these other containable monomer components can be used alone or two or more types can be used in combination. The copolymerization ratio of these other containable monomer components is preferably 30% by weight or less and more preferably 20% by weight or less based on the total of the copolymer.

The ratio of the (meth)acrylic acid alkyl ester monomer component to the entire (meth)acrylic acid ester copolymer is 60 to 100% by weight, and a (meth)acrylic acid ester copolymer containing 70 to 100% by weight of a (meth) acrylic acid ester monomer component based on the total of the copolymer is particularly preferable.

More specifically, a copolymer of 2-ethylhexyl acrylate and acrylic acid is preferable for the (meth)acrylic acid ester copolymer, and the blending ratio is preferably 85:15 to 99:1 (mass ratio).

The (meth)acrylic acid ester copolymer can typically be synthesized by radical polymerization. Examples of polymerization methods include solution polymerization, emulsion polymerization and mass polymerization, and solution polymerization is preferable since it allows the obtaining of satisfactory adhesive properties.

The polymerization reaction comprises adding a radical polymerization initiator at a ratio of about 0.1 to 1% by weight based on the total of the monomer component followed by polymerizing by stirring for several hours to several tens of hours at a temperature of about 40 to 90˚C under nitrogen stream. Furthermore, examples of the polymerization initiator used here include organic peroxides such as benzoyl peroxide or lauroyl peroxide, and azo-based initiators such as azobisisobutyronitrile.

In the patch for nails and/or skin in the present invention, any arbitrary silicone-based pressure-sensitive adhesive ordinarily used in patches for nails and/or skin can be used. For example, the silicone-based pressure-sensitive adhesive described in Japanese Unexamined Patent Publication No. 2006-213650 can be used. Examples of such silicone-based pressure-sensitive adhesives include mixtures or partial condensates of silicone rubber and silicone resin. Examples of silicone rubber include high molecular weight, linear polydiorganosiloxanes having a silicon functional group such as a silanol group on both ends thereof, while examples of silicone resins include polyorganosiloxanes having a branched or mesh structure containing a monofunctional siloxane unit and tetrafunctional siloxane unit, and having silicon function group such as a silanol group or methoxy group in a molecule thereof. More specifically, such silicone rubber include long-chain copolymers of polydimethylsiloxane, while such silicone resins include MQ resins (silicone resins having a three-dimensional structure composed of M unit ($(CH_3)_3SiO_{1/2}$) and Q unit ($SiO_2$)).

Although there are no particular limitations on the composite ratio of silicone rubber/silicone resin that compose the silicone-based pressure-sensitive adhesive, the composite ratio is preferably 30:70 to 60:40 and more preferably 35:65 to 45:55 (mass ratio). In the present invention, examples of particularly preferable composite ratios of silicone rubber/silicone resin include 40/60 (w/w) (BIO-PSA4501, Dow Corning Corp.) and 45/55 (w/w) (BIO-PSA4601, Dow Corning Corp.).

The silicone-based pressure-sensitive adhesive shows pressure-sensitive adhesiveness as a result of silicone functional groups present within a molecule thereof. Examples of organic groups bound to silicone atoms include various types of monovalent hydrocarbon groups such as methyl, ethyl, vinyl and phenyl groups, and pressure-sensitive adhesiveness can be adjusted by selecting the type of substituent. Since the intermolecular distance between polyorganosiloxane molecules which are the major components of silicone-based pressure-sensitive adhesives is large, these adhesives are rich in air permeability and moisture permeability.

Ratio of Antimycotic to Acrylic-Based Pressure-Sensitive Adhesive Layer or Silicone-Based Pressure-Sensitive Adhesive Layer

The ratio of antimycotic incorporated in the acrylic-based pressure-sensitive adhesive layer or silicone-based pressure-sensitive adhesive layer has for an upper limit thereof the amount of antimycotic that can be contained in the formulation in a dissolved state, and can be suitably determined according to the type of antimycotic. In the case of incorporating terbinafine-base as an antimycotic in an acrylic-based pressure-sensitive adhesive, the terbinafine-base is preferably incorporated at 35% by weight or less, more preferably 5 to 35% by weight, even more preferably 15 to 35% by weight, and particularly preferably 20 to 35% by weight based on the total of the acrylic-based pressure-sensitive adhesive layer.

In the case of incorporating terbinafine-base as an antimycotic in a silicone-based pressure-sensitive adhesive, the terbinafine-base is preferably incorporated at 10% by weight or less, more preferably 0.5 to 10% by weight, and particularly preferably 2 to 10% by weight based on the total of the silicone-based pressure-sensitive adhesive.

Other Additives

Fillers, antioxidants and other additives ordinarily used in patches and the like can be contained in the acrylic-based pressure-sensitive adhesive or silicone-based pressure-sensitive adhesive of the patch for nails and/or skin in the present invention in addition to the previously described adhesive components. Specific examples of fillers that can be incorporated include calcium carbonate, magnesium carbonate, silicates, zinc oxide, titanium oxide, magnesium sulfate and calcium sulfate, while specific examples of antioxidants that can be incorporated include butylhydroxytoluene.

An important characteristic in the present invention resides in that a dissolving agent for preventing precipitation of crystals of the drug in the pressure-sensitive adhesive layer, or a permeation enhancer for promoting permeation of the drug into the nails and/or skin is not necessarily required to incorporate. The dissolving agent is a dissolving agent selected from, for example, polyvalent alcohols (such as glycerin, sorbitol, propylene glycol, 1,3-butylene glycol, 1,3-tetramethylene glycol or polyethylene glycol), phenols (such as thymol, safrole, isosafrole, eugenol or isoeugenol), higher alcohols (such as benzyl alcohol, oleyl alcohol, cetyl alcohol, stearyl alcohol, cetostearyl alcohol or octyl dodecanol), ester-based surfactants (such as sesquioleic acid sorbitan, polyoxyethylene hydrogenated castor oil or polyoxyl stearate), fatty acid esters (such as isopropyl myristate, octyldodecyl myristate, oleyl oleate, diethyl phthalate or dibutyl phthalate), and organic acids (such as lactic acid, citric acid, tartaric acid, maleic acid or malic acid). In addition, the permeation enhancer is selected from, for example, fatty acids and fatty acid esters (such as isopropyl myristate, isopropyl palmitate, diisopropyl sebacate, diethyl sebacate, diisopropyl adipate or diethyl adipate), fatty acid amides, fatty alcohols, 2-(2-ethoxyethoxy)-ethanol, glycerol esters, glycerol monolaurate, propylene glycol, polyethylene glycol, unsaturated poly-glycolated glycerides, saturated polyglycerides, $\alpha$-hydroxy acids, dimethyl sulfoxide, decylmethylsulfoxide, pyrrolidones, salicylic acid, lactic acid, dimethylformamide, dimethylacetamide, sodium dodecyl sulfate, phospholipids, oleic acid, oleic acid/2-(2-ethoxyethoxy)ethanol and proteases.

In addition, various types of crosslinking agents can be further added to the pressure-sensitive adhesive layer for the purpose of increasing the cohesive force of the acrylic-based pressure-sensitive adhesive used in the pressure-sensitive adhesive layer of the patch for nails and/or skin in the present invention. Examples of crosslinking agents include multifunctional isocyanate compounds, multifunctional epoxy compounds and polyvalent metal salts. More specifically, polyisocyanate (such as Coronate HL (hexamethylene diisocyanate HDI-TMP adduct, Nippon Polyurethane Industry Co., Ltd.) is preferable.

On the other hand, various types of silicone fluids can also be added for the purpose of improving the adhesiveness of the silicone-based pressure-sensitive adhesive used in the pressure-sensitive adhesive layer of the patch for nails and/or skin in the present invention. Examples of such silicone fluids able to be incorporated include polydimethylsiloxane fluids in which both ends of the molecular chain are blocked with trimethylsiloxy groups, copolymer fluids of dimethylsiloxane and methylphenylsiloxane, copolymer fluids of dimethylsiloxane and methylvinylsiloxane, copolymer fluids of dimethylsiloxane and diphenylsiloxane, copolymer fluids of dimethylsiloxane and methyl(2-phenylpropyl)siloxane, copolymer fluids of dimethylsiloxane, methyl(2-phenylpropyl) siloxane and methyloctylsiloxane, and polyoxyalkylene-modified polydimethylsiloxane fluids.

## Production Process of Patch in the Present Invention

In producing the patch in the present invention, a mixture of an active ingredient in the form of an antimycotic, a pressure-sensitive adhesive (acrylic-based pressure-sensitive adhesive or silicone-based pressure-sensitive adhesive), and as desired, another additive, can be coated onto a suitable release liner and a suitable support can be laminated thereon followed by cutting to a suitable size as necessary to ultimately obtain a finished product.

A backing layer able to be used for the patch in the present invention can be suitably selected according to the purpose of use by taking into consideration the fit to the affected area and the ease of adhering at the time of adhesion as well as in consideration of such factors as flexibility, stretchability and thickness. Examples of backing layers such include paper such as impregnated paper, coated paper, wood-free paper, kraft paper, Japanese paper or glassine paper; plastic film such as polyester film, polyethylene film, polypropylene film, polyvinyl chloride film, polycarbonate film, polyurethane film or cellophane film; foam; fabric base materials such as non-woven fabric, woven fabric or knitted fabric including polyester fibers, polyethylene fibers or polypropylene fibers; and, laminates thereof. Among these, olefin-based plastic films such as polyethylene film are preferable in terms of stretchability and ease of use.

The thickness of the backing layer used in the case of a plastic film is preferably 1 to 200 $\mu$m and more preferably 30 to 100 $\mu$m.

The release liner used in the patch in the present invention can be suitably selected according to the purpose of use in consideration of factors such as ease of release from the pressure-sensitive adhesive layer, air permeability, moisture permeability and flexibility. A film having a thickness of about 10 to 200 $\mu$m comprising of a polymer material such as polyethylene, polypropylene or polyester is used preferably, and a film can also be used in which the film surface has been subjected to silicone treatment or fluorocarbon treatment in order to enhance releasability.

A process ordinarily used to produce patches can be suitably used to produce the patch in the present invention. More specifically, a production process indicated below can be mentioned.

In the case of using an acrylic-based pressure-sensitive adhesive having a (meth)acrylic acid ester copolymer for the base, the (meth)acrylic acid ester copolymer is first synthesized by, for example, solution polymerization as previously described, and a patch is produced by a solution coating method using the resulting (meth)acrylic acid ester copolymer solution.

In a solution coating method, a solution is first prepared by adding a synthesized (meth)acrylic acid ester copolymer (pressure-sensitive adhesive) solution, antimycotic such as a terbinafine-base (active ingredient), and as desired, a crosslinking agent or other additives. An organic solvent as a diluent can then be added to this solution to adjust to a suitable concentration.

Examples of organic solvents used here include n-hexane, toluene, ethyl acetate, acetone and methyl ethyl ketone. In the case of adding these organic solvents as a diluent, a solution is prepared that contains the (meth)acrylic acid ester copolymer preferably at 10 to 50% by weight and more preferably at 20 to 40% by weight based on the mass weight of the entire solution, using these organic solvents.

In addition, the amount of antimycotic incorporated in the solution can be suitably determined according to the type of antimycotic and the amount of (meth)acrylic acid ester copolymer.
The amounts of crosslinking agent and other additives can be suitably determined according to the amounts of each ingredient.

Next, the solution (diluted solution) containing each component is stirred to uniformly dissolve and disperse each component. The solution obtained in this manner is then uniformly coated onto, for example, a release liner (silicone-treated polyester film) using a coating machine such as a knife coater, comma coater or reverse coater. The coated amount of the solution can be suitably determined according to the thickness of the desired acrylic-based pressure-sensitive adhesive layer or silicone-based pressure-sensitive adhesive layer, type of pressure-sensitive adhesive used, type of antimycotic and amounts thereof. For example, in the case of a solution containing 20% by weight of (meth) acrylic acid ester copolymer based on the weight of the entire solution, and 10% by weight of terbinafine-base based on the mass weight of the entire solution, then the solution is preferably coated to a thickness of 10 to 200 $\mu$m and more preferably 20 to 100 $\mu$m.

After coating the solution, the organic solvent is vaporized by holding for about 30 seconds to 10 minutes in a dry heat atmosphere maintained at a temperature of about 40 to 130°C. Drying conditions are suitably selected according to the type of organic solvent used and thickness of the coated pressure-sensitive adhesive.

An antimycotic patch is obtained by laminating a support onto the surface of the pressure-sensitive adhesive layer obtained in the manner described above. A release liner may be laminated onto the surface of the pressure-sensitive adhesive layer after coating the pressure-sensitive adhesive layer on the backing layer depending on the type of backing layer.

Examples

The following provides a more detailed explanation in the present invention by listing examples thereof, but the present invention is not limited to these examples. In the examples, "%" and "part(s)" mean "% by weight" and "part (s) by mass", respectively. Furthermore, in the following examples and comparative examples, any description of terbinafine refers to terbinafine-base.

Example 1

A patch was obtained according to the composition and production process described below.

| | | |
|---|---|---|
| 1. | Terbinafine[*1] | 20.0% |
| 2. | Acrylic-based pressure-sensitive adhesive[2*] | 79.7% (solid content) |
| 3. | Polyisocyanate[*3] | 0.3% |

*1: purchased from Kaneda Corp.
*2: Obtained by ordinary solution polymerization of 96% 2-ethylhexyl acrylate and 4% acrylic acid using 0.5 parts of polymerization initiator in the form of lauryl peroxide in ethyl acetate at a concentration of 33%. Subsequent values for the pressure-sensitive adhesive indicate the solid content thereof.
*3: Coronate HL (Nippon Polyurethane Industry Co.)

(Production Process)

After weighing out each of the above ingredients, the total solid content was adjusted to 25% in ethyl acetate and stirred to uniformity. The solution was coated onto a 75 $\mu$m-thick PET (polyethylene terephthalate) film and subjected to silicone treatment on one side thereof (Filmbyna 75E-0010 No. 23, Fujimori Kogyo Co., Ltd.) so that the thickness of the pressure-sensitive adhesive layer at the time of coating was 30 $\mu$m thick, followed by drying at 110°C for 3 minutes. Next, a 25 $\mu$m-thick PET film (Lumirror S10, Toray Industries, Inc.) was laminated onto one side of the pressure-sensitive adhesive layer to obtain a patch.

Examples 2 to 6

Patches were obtained using the compositions shown in Table 1 and the same production process as Example 1, and making the content of terbinafine 30.0%, 32.0%, 35.0%, 5% or 1%.

Table1

|  | Example 2 | Example 3 | Example 4 | Example 5 | Example 6 |
|---|---|---|---|---|---|
| Terbinafine [*1] | 30.0% | 32.0% | 35.0% | 5% | 1% |
| Acrylic-based pressure sensitive adhesive [*2] | 69.6% | 67.6% | 64.5% | 94.7% | 98.7% |
| Polyisocyanate [*3] | 0.4% | 0.4% | 0.5% | 0.3% | 0.3% |
| *1: Same as that used in Example 1<br>*2: Same as that used in Example 1<br>*3: Coronate HL (Nippon Polyurethane Industry Co., Ltd.) | | | | | |

Example 7

A patch was obtained using the following composition and production process while making the terbinafine content 10.0% and using a silicone-based pressure-sensitive adhesive for the base.

|  |  |  |
|---|---|---|
| 1. | Terbinafine[*1] | 10.0% |
| 2. | Acrylic-based pressure-sensitive adhesive[*] | 90.0% |

*1: Same as that used in Example 1
*4: BIO-PSA4501 (Dow Corning Corp.)

A mixture of polydimethylsiloxane and MQ resin (branched polysiloxane having M unit ($(CH_3)_3SiO_{1/2}$) and Q unit ($SiO_2$)) at the a composite ratio of 60:40 in heptane at a concentration of 63% was polymerized by ordinary solution polymerization. Subsequent values for the pressure-sensitive adhesive indicate the solid content thereof.

(Production Process)

Each of the above ingredients was weighed and stirred to uniformity. The solution was coated onto a 75 µm-thick PET (polyethylene terephthalate) film and subjected to silicone treatment on one side thereof (Filmbyna 161-8066 FN-75, Fujimori Kogyo Co., Ltd.) so that the thickness of the pressure-sensitive adhesive layer at the time of coating was 30 µm thick, followed by drying at 110˚C for 3 minutes.
Next, a 25 µm-thick PET film (Lumirror S10, Toray Industries, Inc.) was laminated onto one side of the pressure-sensitive adhesive layer to obtain a patch.

Comparative Example 1

A patch was obtained using the components indicated below and the same production process as Example 1 and using terbinafine hydrochloride for the drug at a concentration of 10.0%.

|  |  |  |
|---|---|---|
| 1. | Terbinafine hydrochloride [*5] | 10.0% |
| 2. | Acrylic-based pressure sensitive adhesive[*2] | 89.8% |
| 3. | Polyisocyanate[*3] | 0.2% |

*2: Same as that used in Example 1
*3: Same as that used in Example 1
*5: Purchased from Kaneda Corp.

Comparative Example 2

A patch was obtained using the components indicated below and the same production process as Example 1 and adding polyethylene glycol as a permeation enhancer.

|  |  |  |
|---|---|---|
| 1. | Terbinafine hydrochloride [*5] | 10.0% |
| 2. | Acrylic-based pressure sensitive adhesive[*2] | 39.8% |

(continued)

| 3. | Polyisocyanate[*3] | 0.2% |
|---|---|---|
| 4. | Polyethylene glycol #400 | 50.0% |

*2: Same as that used in Example 1
*3: Same as that used in Example 1
*5: Same as that used in Comparative Example 1

Comparative Example 3

A patch was obtained using the components indicated below and the same production process as Example 1 and adding triacetin as a permeation enhancer.

| 1. | Terbinafine hydrochloride[*5] | 10.0% |
|---|---|---|
| 2. | Acrylic-based pressure sensitive adhesive[2*] | 69.7% |
| 3. | Polyisocyanate[*3] | 0.3% |
| 4. | Triacetin | 20.0% |

*2: Same as that used in Example 1
*3: Same as that used in Example 1
*5: Same as that used in Comparative Example 1

Comparative Example 4

A patch was obtained using the components indicated below and the same production process as Example 1 and using a rubber-based pressure-sensitive adhesive for the base.

| 1. | Terbinafine[*1] | 10.0% |
|---|---|---|
| 2. | Rubber-based pressure sensitive adhesive[*6] | 90.0% |

*1: Same as that used in Example 1
*6: Pressure-sensitive adhesive in the form of a rubber-based pressure-sensitive adhesive (59.6% styrene-isoprene-styrene block copolymer (SIS5002, JSR Corp.), 29.8% hydrogenated rosin ester resin (Pinecrystal KE311, Arakawa Chemical Industries, Ltd.) and 0.6% dibutylhydroxytoluene (BHT-F, Takeda-Kirin Foods Corp.))

(Production Process)

After weighing each of the above components, the total solid content was adjusted to 50% in toluene/hexane solution (composite ratio: 2/1) and stirred to uniformity. The solution was coated onto a 75 μm-thick PET (polyethylene terephthalate) film and subjected to silicone treatment on one side thereof (Filmbyna 75E-0010 No. 23, Fujimori Kogyo Co., Ltd.) so that the thickness of the pressure-sensitive adhesive layer at the time of coating was 30 μm thick, followed by drying at 110˚C for 3 minutes.
Next, a 25 μm-thick PET film (Lumirror S10, Toray Industries, Inc.) was laminated onto one side of the pressure-sensitive adhesive layer to obtain a patch.

Comparative Example 5

A patch was obtained using the components indicated below and the same production process as Example 1 and making the terbinafine content 40.0%

| 1. | Terbinafine[*1] | 40.0% |
|---|---|---|
| 2. | Acrylic-based pressure sensitive adhesive[2*] | 59.7% |

(continued)

| | | |
|---|---|---|
| 3. | Polyisocyanate[*3] | 0.3% |

*1: Same as that used in Example 1
*2: Same as that used in Example 1
*3: Same as that used in Example 1

Comparative Example 6

A patch was obtained using the components indicated below and the same production process as Example 1, making the terbinafine content 20.0% and using a silicone-based pressure-sensitive adhesive for the base.

| | | |
|---|---|---|
| 1. | Terbinafine[*1] | 20.0% |
| 2. | Silicone-based pressure sensitive adhesive[*4] | 80.0% |

*1: Same as that used in Example 1
*4: Same as that used in Example 7

[Evaluation Test]

The patches of each of the examples and comparative examples were tested using the evaluation test methods indicated below.

Test Example 1 - Terbinafine Patch Crystal

Precipitation Test

The patches produced in each of the examples and comparative examples were heat-sealed in a composite film pouch using aluminum foil as a base thereof (PET 12 $\mu$m/PE 15 $\mu$m/Al 9 $\mu$m/PE 25 $\mu$m). The pouches were then stored at room temperature (about 20°C) and low temperature (5°C) followed by visual confirmation of the status of the antimycotic in the formulation after aging for up to 6 months.
As is clear from Table 2, in the formulation of Comparative Examples 1 to 3, crystals of terbinafine hydrochloride were visually confirmed to be dispersed throughout the pressure-sensitive adhesive layer of the formulation at the time of production. In the formulation of Comparative Examples 5 and 6, precipitation of crystals was observed after 2 months of cold storage. On the other hand, precipitation of crystals was not observed and the preparations were confirmed to be stable even after room temperature and cold storage for 6 months for each of the preparations of Examples 1 to 7 and Comparative Example 4.

Test Example 2 - Terbinafine Patch Adhesion Test

Adhesion was evaluated according to finger tack based on the evaluation criteria indicated below for each of the formulation of Examples 1 to 7 and Comparative Examples 1 to 6.
○: Good △: Suitable ✕: Unsuitable
Moreover, the formulation were also evaluated for residual pressure-sensitive adhesive (residual adhesive).
As is clear from Table 2, the formulation of Example 1 to 7 exhibited sufficient pressure-sensitive adhesiveness and adhesion as a pressure-sensitive adhesive without any residual adhesive. Although the formulation of Comparative Example 4 was observed to be free of residual adhesive, it did not exhibit pressure-sensitive adhesiveness capable of withstanding long-term adhesion. The preparations of Comparative Examples 1 to 3 and 5 and 6 lacked adhesion due to dispersion of crystals in the pressure-sensitive adhesive or the precipitation of crystals after aging, and did not exhibit the function as a patch. In the formulations of Comparative Examples 2 and 3, residual adhesive was observed possibly due to the addition of oily components.
In addition, adhesion to nails of the feet was evaluated by wrapping the formulation around the ends of the toenails of three volunteers each so as to adhere thereto followed by evaluating the adhered state after 3 days of adhesion as to whether the formulations remained adhered (good) or came off. As is clear from Table 2, the preparations of Examples 1 to 7 demonstrated superior adhesion for a long period of time. The formulation of Comparative Example 4 was observed to come off on the first day after adhering to the toenail.
On the basis of the above results, in the case of formulations using a rubber-based pressure-sensitive adhesive for the base, practical pressure-sensitive adhesiveness is unable to be obtained even for a terbinafine content of 10%, while acrylic-based and silicone-based pressure-sensitive adhesives were found to have superior practicality.

In addition, in the case of using terbinafine hydrochloride (logKo/w value: 0.87 (from the interview form provided with 125 mg Lamisil Tablets)) even with an acrylic-based pressure-sensitive adhesive as in Comparable Examples 1 to 3, since crystals of the drug precipitate and adhesion is unsuitable, in order to add a high concentration of drug, it was recognized to be necessary to remove the salt to convert to the free form and use a drug having a logko/w value of 4 or more in order to maintain a high concentration of drug.

Moreover, if terbinafine is contained in an acrylic-based pressure-sensitive adhesive at up to 40% or in a silicone-based pressure-sensitive adhesive at up to 20% as in Comparative Examples 5 and 6, the drug is unable to be retained and adhesion becomes unsuitable. Thus, the preferable upper limit concentrations were recognized to be 35% for an acrylic-based pressure-sensitive adhesive and 10% for a silicone-based pressure-sensitive adhesive.

Test Example 3 - Terbinafine Nail Penetration Test

The nail permeation of the patches obtained in Examples 1, 2, 4, 5 and 7 and Comparative Example 4 was confirmed using human nails. The test was carried out by adhering the patches of Examples 1, 2, 4, 5 and 7 and Comparative Example 4 to human nails followed by adhering a placebo tape produced according to the production process of Example 1 having the composition indicated below to a receiver side on the opposite side of the nail. The placebo tape was changed on days 1, 2, 3, 4, 9, 14 and 17 from the start of the test through day 17.

Moreover, the patch of Example 2 was adhered for 42 consecutive days, the upper and lower layers of the nail were sliced crosswise into two sections following completion of testing and the concentration of terbinafine in the nail was measured for each layer.

After recovering the placebo tape and extracting the terbinafine, and dissolving the nail in an aqueous sodium hydroxide solution followed by extracting the terbinafine, the cumulative amount of terbinafine that permeated through the nail and the amount of terbinafine remaining in the nail were calculated by HPLC. The test was repeated three times on each specimen to obtain an average value.

<Placebo Tape>

| | | |
|---|---|---|
| 1. | Acrylic-based pressure-sensitive adhesive[*2] | 99.9% |
| 2. | Polyisocyanate[*3] | 0.1% |
| *2: Same as that used in Example 1 | | |
| 3: Same as that used in Example 1 | | |

As is clear from Table 3 and Fig. 1, the formulations of Examples 1, 2, 4 and 5 using an acrylic-based pressure-sensitive adhesive demonstrated drug concentration-dependent penetrated amounts and nail residual amounts of terbinafine, and even in the formulation of Example 5 containing 5% terbinafine, the terbinafine was determined to have penetrated the nail, although in only a trace amount, even on day 1 of adherence. Moreover, in each of the examples, the amounts of terbinafine in the nail on day 17 of adherence demonstrated values 40 to 500 times greater than that of $0.78\pm0.3$ $\mu$g/g in the nails ("Clinical and Pharmacokinetic Study of Orally Administered Terbinafine for Tinea Unguium", Matsumoto, T. et al., Nishi Nihon Hifuka, Vol. 56, No. 2, 1994) during oral administration of terbinafine hydrochloride at 125 mg/day for 24 weeks.

However, trichophytons are known to be present in the nail bed beneath nails, a concentration gradient exists by passive diffusion of a drug for the distribution of drug concentration in nails, and this is presumed to cause a decrease in drug concentration the greater the depth beneath nails. In actuality, the drug concentration in the lower portion of the nail of Example 2, in which a patch was adhered for 42 consecutive days, was 2.51 $\mu$g/g, corresponding to a decrease in concentration of about 3.2 times greater than the concentration of 0.78 $\mu$g/g during oral administration. Since the amount of drug transferred to the entire nail of Example 2 is 362.79 $\mu$g/g, a concentration of 110 $\mu$g/g was judged to be required for the amount of drug transferred to the nail. Thus, a permeation is preferable in which the concentration of terbinafine incorporated in the formulation is 20% or more.

The formulation of Example 7, which used a silicone-based pressure-sensitive adhesive, also exhibited sufficient nail permeation and nail retention, and the amount of drug retained in the nail was 250 times or more greater than in the case of oral administration.

On the other hand, Comparative Example 4, which used a rubber-based pressure-sensitive adhesive, demonstrated remarkably lower penetration nail penetration than Example 7 containing the same amount of drug. In addition, adhesion to the nail at the time of testing was poor in comparison with each of the examples and unsuitable for long-term adherence.

Test Example 4 - Terbinafine Patch Antimicrobial Activity Test

A section of human nail was punched out to a diameter of 5 mm and sterilized by immersing in 70% ethanol solution for 60 minutes followed by allowing to stand for 1 day in a constant temperature chamber[*7] at 32°C and 50% RH to acclimate the nail prior to use in the test.

The medium was prepared by placing 1.0 g of dipotassium phosphate, 0.025 g of calcium sulfate, 0.025 g of calcium chloride, 7.5 g of agar and 491 ml of distilled water in a 500 mL Erlenmeyer flask, heating at 95°C for 30 minutes, shaking to uniformity and dispensing 5 mL each into test tubes followed by further subjecting to high-pressure steam sterilization at 121°C for 15 minutes with a high-pressure steam sterilizer[*8] and then maintaining the medium at 50°C in a constant temperature chamber[*7]

Separate from the above, *Trichophyton mentagrophytes* (NBRC 32410) was cultured in a constant temperature chamber[*7] at 28°C for 14 days on Sabaraud agar slant medium (Nippon Becton Dickinson Co., Ltd.) followed by layering thereon 4 ml of sterile physiological saline containing 0.05% of polyoxyethylene sorbitan monooleate and using the liquid obtained by scratching off the surface of the slant with a key-shaped platinum wire for use as a conidia suspension after filtering with sterile gauze. The number of conidia were counted with a cell counting chamber and adjusted to a count of $1 \times 10^7$ cells/mL in sterile physiological saline. 50 μL of conidia suspension adjusted to $1 \times 10^7$ cells/mL and 5 mL of medium maintained at 50°C in a constant temperature chamber[*7] were placed in a sterile Petri dish, immediately mixed and diluted by moving the Petri dish followed by solidifying the medium by cooling to room temperature to prepare the test medium.

One piece each of sterilized nail was placed on the test medium and cultured in a constant temperature chamber[*7] at 28°C for 7 days. The nails were removed after 7 days, microorganisms adhered to the periphery were wiped gently with sterile sanitary cotton moistened with sterile physiological saline, and the nails were dried gently by allowing to stand for about 1 hour. Test materials punched out to a diameter of 4 mm (Examples 1, 2, 5, 6 and placebo tape) were adhered to the nails, placed on medium not containing *Trichophyton mentagrophytes* and cultured again in a constant temperature chamber[*7] at 28°C for 7 days. Evaluations were made by visually observing the nails and surrounding medium based on five levels of evaluation criteria consisting of 0: no growth of *Trichophyton mentagrophytes,* 0.5: slight growth, 1: definite growth, 2: growth beyond the periphery of the nail and 3: growth covering the entire nail.

As shown in Table 4, superior antimicrobial activity was confirmed for all formulations with terbinafine concentrations of 5% or more.

*7: Constant temperature chamber ICB-151L (Iwaki Co., Ltd.)
*8: High-pressure steam sterilizer CLS-40S (Alp Co., Ltd.)
Table 2

| Item | Example | | | | | | |
|------|---|---|---|---|---|---|---|
|  | 1 | 2 | 3 | 4 | 5 | 6 | 7 |

| Terbinafine | 20.0% | 30.0% | 32.0% | 35.0% | 5.0% | 1.0% | 10.0% |
|---|---|---|---|---|---|---|---|
| Terbinafine hydrochloride | - | - | - | - | - | - | - |
| Pressure-sensitive adhesive | Acrylic | Acrylic | Acrylic | Acrylic | Acrylic | Acrylic | Silicone |
| Status of drug | Dissolved | Dissolved | Dissolved | Dissolved | Dissolved | Dissolved | Dissolved |
| Crystal precipitation | No | No | No | No | No | No | No |
| Adhesion (finger tack) | ○ | ○ | ○ | ○ | ○ | ○ | ○ |
| Residual adhesive | No | No | No | No | No | No | No |
| Toenail adhesion | Good | Good | Good | Good | Good | Good | Good |

| Item | Comparative Examples | | | | | |
|---|---|---|---|---|---|---|
|  | 1 | 2 | 3 | 4 | 5 | 6 |
| Terbinafine | - | - |  | 10.0% | 40.0% | 20.0% |
| Terbinafine hydrochloride | 10.0% | 10.0% | 10.0% | - | - | - |
| Dissolving agent or enhancer | - | PEG #400 | Triacetin | - | - | - |
| Type of pressure-sensitive adhesive | Acrylic | Acrylic | Acrylic | Rubber | Acrylic | Silicone |
| Status of drug | Dispersed | Dissolved | Dispersed | Dissolved | Dissolved | Dissolved |
| Crystal precipitation | Yes | Yes | Yes | No | Yes after 2 months | Yes after 2 months |
| Adhesion (finger tack) | × | × | × | △ | × | × |
| Residual adhesive | No | Yes 1) | Yes 1) | No | No | No |
| Toenail adhesion | - | - | - | Came off | - | - |

○: Good    △:Suitable    ×:Unsuitable

1) Adhesion remained on skin due to occurrence of cohesive failure.

Table 3

| Item | Example 1 | Example 2 | Example 4 | Example 5 | Example 7 | Comparative Examples 4 |
|---|---|---|---|---|---|---|
| Terbinafine concentration | 20.0% | 30.0% | 35.0% | 5.0% | 10.0% | 10.0% |
| Type of pressure-sensitive adhesive | Acrylic | Acrylic | Acrylic | Acrylic | Silicone | Rubber |
| Cumulative nail penetration 0 | 0 | 0 | 0 | 0 | 0 | 0 |
| Day 1 | 0.04 | 0.06 | 0.07 | 0.09 | 0.21 | 0.02 |
| Day 2 | 0.15 | 0.22 | 0.26 | 0.11 | 0.69 | 0.18 |
| Day 3 | 0.34 | 0.45 | 0.54 | 0.21 | 1.22 | 0.27 |
| Day 4 | 0.51 | 0.69 | 0.91 | 0.23 | 1.80 | 0.40 |
| Day 9 | 1.48 | 2.06 | 2.89 | 0.43 | 4.86 | 1.09 |
| Day 14 | 2.83 | 3.92 | 5.02 | 0.61 | 7.65 | 1.89 |
| Day 17 | 3.56 | 5.03 | 6.51 | 0.74 | 9.38 | 2.39 |
| Cumulative nail drug concentration ($\mu$g/g) [SE] | 108.92 [32.34] | 362.79 [125.12] | 271.60 [29.98] | 32.14 [16.13] | 224.50 [11.45] | 49.70 [12.78] |

Table 4

| Item | Placebo | Example 1 | Example 2 | Example 5 | Example 6 |
|---|---|---|---|---|---|
| Terbinafine | - | 20.0% | 30.0% | 5.0% | 1.0% |
| Terbinafine hydrochloride | - | - | - | - | - |
| Pressure-sensitive adhesive | Acrylic | Acrylic | Acrylic | Acrylic | Acrylic |
| Degree of elimination of *Trichophyton mentagrophytes* | 2 | 0 | 0 | 0 | 1 |

[INDUSTRIAL APPLICABILITY]

The nail and/or skin patch for preventing or treating mycoses of the present invention is capable of demonstrating sufficient effects for treatment of mycoses of the nails and skin, and is particularly useful for the treatment of superficial mycoses such as Athlete's foot and tinea.

**Claims**

1. A nail and/or skin patch for preventing or treating mycoses, which contains an antimycotic, having an octanol/water partition coefficient in the form of a logKo/w value of 4 or more, in a dissolved state in an acrylic-based pressure-sensitive adhesive layer or silicone-based pressure-sensitive adhesive layer.

2. The patch according to claim 1, wherein the antimycotic is terbinafine-base.

3. The patch according to claim 2, wherein the terbinafine-base is contained at a ratio of 5 to 35% by weight based on the total weight of the acrylic-based pressure-sensitive adhesive layer.

4. The patch according to claim 2, wherein the terbinafine-base is contained at a ratio of 15 to 35% by weight based on the total weight of the acrylic-based pressure-sensitive adhesive layer.

5. The patch according to claim 2, wherein the terbinafine-base is contained at a ratio of 20 to 35% by weight based on the total weight of the acrylic-based pressure-sensitive adhesive layer.

6. The patch according to claim 2, wherein the terbinafine-base is contained at a ratio of 0.5 to 10% by weight based on the total weight of the silicone-based pressure-sensitive adhesive layer.

7. The patch according to claim 2, wherein the terbinafine-base is contained at a ratio of not more than 2 to 10% by weight based on the total weight of the silicone-based pressure-sensitive adhesive layer.

8. The patch according to claim 1, wherein the antimycotic is a blend of a terbinafine acid addition salt and a base.

9. The patch according to claim 8, wherein the base is selected from the group consisting of potassium hydroxide, sodium hydroxide, calcium hydroxide, magnesium hydroxide, sodium carbonate, sodium hydrogencarbonate, phosphates, borates, acetates, ammonia, dialkylamines, trialkylamines, tris(hydroxymethyl) aminomethane, monoethanolamine, diethanolamine, triethanolamine, isopropanolamine, diisopropanolamine and triisopropanolamine.

10. The patch according to any of claims 1 to 9 for treatment of tinea unguium.

11. A nail and/or skin patch for preventing or treating tinea unguium containing terbinafine-base in an acrylic-based pressure-sensitive adhesive layer wherein the terbinafine-base is contained in a dissolved state at a ratio of 20 to 35% by weight based on the total weight of the acrylic-based pressure-sensitive adhesive layer.

12. The patch according to any of claims 1 to 11, which does not contain neither drug dissolving agent nor permeation enhancer of drug.

# Fig. 1

<table>
<tr><td colspan="2" align="center">**INTERNATIONAL SEARCH REPORT**</td><td colspan="2">International application No.<br>PCT/JP2008/053085</td></tr>
</table>

A. CLASSIFICATION OF SUBJECT MATTER
*A61K9/70*(2006.01)i, *A61K31/137*(2006.01)i, *A61K47/32*(2006.01)i, *A61P31/10*
(2006.01)i

According to International Patent Classification (IPC) or to both national classification and IPC

B. FIELDS SEARCHED

Minimum documentation searched (classification system followed by classification symbols)
A61K9/70, A61K31/137, A61K47/32, A61P31/10

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2005-501885 A  (TROMMSDORFF ARZNEIMITTEL<br>GMBH & CO KG, DE),<br>20 January, 2005 (20.01.05),<br>Full text; particularly, example 4<br>& WO 2003/020250 A1    & EP 1423102 A1<br>& US 2004/265362 A1 | 1-12<br>1-12 |
| X<br>Y | JP 10-330247 A  (POLA CHEM IND INC.),<br>15 December, 1998 (15.12.98),<br>Full text; particularly, example 6<br>(Family: none) | 1-12<br>1-12 |
| Y | ALBERTI, I. et al, In vivo assessment of<br>enhanced topical delivery of terbinafine to<br>human stratum corneum, J Control Release,<br>2001, Vol.71, No.3, p.319-27, full text,<br>particularly, Abstract | 1-12 |

☒  Further documents are listed in the continuation of Box C.          ☐    See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>11 April, 2008 (11.04.08) | Date of mailing of the international search report<br>22 April, 2008 (22.04.08) |
|---|---|
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2008/053085 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2003-525641 A  (WATSON PHARM INC.),<br>02 September, 2003 (02.09.03),<br>Full text; particularly, example III<br>& WO 99/40955 A2        & EP 1399145 A2<br>& US 6727401 B1 | 1-12 |
| A | JP 2002-363070 A  (YUTOKU YAKUHIN KOGYO KABUSHIKI KAISHA),<br>18 December, 2002 (18.12.02),<br>Full text; particularly, example 17<br>(Family: none) | 1-12 |
| E,A | WO 2007/069662 A1  (NITTO DENKO CORP.),<br>21 June, 2007 (21.06.07),<br>Full text; particularly, comparative examples 11, 12<br>& JP 2007-186500 A | 1-12 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002363070 A
- JP 2003525641 A
- JP 2005501885 A
- JP 2006213650 A

**Non-patent literature cited in the description**

- **Tanojo H. et al.** In vitro human barrier modulation by topical application of fatty acids. *Skin Pharmacol. Appl. Skin Physiol.,* 1998, vol. 11 (2
- **Kanikkannan N. et al.** *Skin permeation enhancement effect and skin irritation of saturated fatty alcohols,* 2002, vol. 6
- **Matsumoto, T. et al.** Clinical and Pharmacokinetic Study of Orally Administered Terbinafine for Tinea Unguium. *Nishi Nihon Hifuka,* 1994, vol. 56 (2